# EUROPÄISCHE PATENTSCHRIFT

(11) **EP 1 579 779 B1**
(45) Veröffentlichungstag und Bekanntmachung des Hinweises auf die Patenterteilung: **18.10.2006**
(21) Anmeldenummer: 04006783.7
(22) Anmeldetag: 22.03.2004
(51) Int. Cl.: A44B 18/00

(54) **Verbundstoff für Klettverschlüsse, insbesondere Windelverschlüsse**
Multi-layer fabric for hook fastener, especially for diaper fastener
Etoffe multi-couches pour fermetures de type Velcro, en particulier pour fermetures de couches

(43) Veröffentlichungstag der Anmeldung: 28.09.2005
(73) Patentinhaber: Nordenia Deutschland Gronau GmbH, 48599 Gronau (DE)
(72) Erfinder: Baldauf, Georg, 48366 Laer (DE); Dr.Thomas Alexander Horn, 95719 Hofheim (DE)
(74) Vertreter: Albrecht, Rainer Harald

(56) Entgegenhaltungen:
- EP-A- 0 233 364
- EP-A- 0 777 006
- FR-A- 2 846 346
- GB-A- 1 438 721

## Beschreibung

Die Erfindung betrifft einen Verbundstoff für Klettverschlüsse, insbesondere Windelverschlüsse, mit einer Trägerfolie und einem auf die Trägerfolie aufkaschierten textilen Substrat, welches oberflächliche, zur Verbindung mit Kletthaken geeignete Schlaufen aufweist.

Der Verbundstoff bildet den weiblichen Teil eines Klettverschlusses. Bei Verwendung an Windeln werden Streifen des Verbundstoffes auf dem vorderen Bündchenbereich der Windeln angebracht. Mit Verschlussbändern, die seitlich an den Windeln befestigt sind und an ihrem freien Ende Kletthaken aufweisen, wird der Klettverschluss vervollständigt. Klettverschlüsse können mehrfach geöffnet und verschlossen werden, ohne dass dadurch die Funktionalität des Verschlusses leidet. Im Gegensatz zu Klebeverschlüssen sind Klettverschlüsse unempfindlich gegenüber Kontakt mit Hautcremes oder Puder.

An einem Verbundstoff für Klettverschlüsse an Wegwerfprodukten, z. B. Babywindeln, werden mehrere Anforderungen gestellt. Das textile Substrat soll ein möglichst geringes Flächengewicht aufweisen, damit es kostengünstig gefertigt werden kann. Es soll durchscheinend sein, damit die zumeist bedruckte Oberfläche der Trägerfolie sichtbar ist. Ferner muss das textile Substrat trotz eines geringen Flächengewichtes eine ausreichende Verhakung mit Kletthaken des zugeordneten Verschlussbandes gewährleisten. Erforderlich ist eine ausreichende Zahl von freibeweglichen Schlaufen, deren Funktion durch eine Verklebung von Trägerfolie und textilem Substrat nicht beeinträchtigt werden darf. Um eine funktionssichere Verklebung des textilen Substrats auf der Trägerfolie zu gewährleisten, muss ein ausreichend dicker Klebstofffilm aufgetragen werden. Wenn das textile Substrat und die Trägerfolie in einem Kaschierwerk mittels eines Walzenpaares aufeinander gepresst werden, sinken die Fasern des textilen Substrats in den Klebstofffilm ein und werden von dem Klebstofffilm umschlossen. Nach Aushärtung des Klebstofffilmes sind die Garne des Textils zwar sicher auf der Trägerfolie verankert, jedoch bergen hohe Klebstoffmengen die Gefahr, dass die Schlaufen, die für die Funktion des Hakenverschlusses nötig sind, mit verklebt werden und dadurch ihre Funktionsfähigkeit verlieren. Dies macht sich in einer unzureichenden Klettwirkung bemerkbar.

Aus EP 0 777 006 B1 ist ein Verbundstoff für Klettverschlüsse mit den eingangs beschriebenen Merkmalen bekannt. Das textile Substrat besteht aus einem Gelege aus Kett- und Schussfäden und wirktechnisch mit dem Gelege verbundenen Schlaufen. Das textile Substrat ist mit der Trägerfolie verklebt. Die Schlaufen sind so groß bemessen, dass sie auf den von dem Grundgelege gebildeten Maschen aufliegen. Durch die Bemessung der Schlaufen soll erreicht werden, dass die Schlaufen nicht mit Klebstoff in Verbindung kommen und ihre Funktionsfähigkeit behalten. Das vorstehend erläuterte Problem, bei Verwendung eines offenen textilen Substrats sowohl eine gute Klettwirkung als auch eine hohe Verbundfestigkeit zwischen Trägerfolie und textilem Substrat sicherzustellen, ist jedoch noch nicht in vollem Umfange gelöst. Insbesondere die Verbindung zwischen Trägerfolie und textilem Substrat ist noch verbesserbungsbedürftig.

Der Erfindung liegt die Aufgabe zugrunde, in einem Verbundstoff für Klettverschlüsse die Verbindung zwischen Trägerfolie und textilem Substrat so auszubilden, dass sich bei einer hohen Verbundfestigkeit eine verbesserte Klettwirkung im Vergleich zum Stand der Technik einstellt.

Die Aufgabe wird erfindungsgemäß dadurch gelöst, dass die Trägerfolie und das textile Substrat nicht vollflächig verbunden sind. Die Fläche, an der die Trägerfolie mit dem textilen Substrat verbunden ist, beträgt vorzugsweise 20 bis 80 % der kaschierten Folienfläche. Erfindungsgemäß liegen Trägerfolie und das textile Substrat auf einem Teil der Kaschierfläche lose aufeinander auf. In diesen Bereichen, in denen Trägerfolie und das Substrat nicht miteinander verbunden sind, können sich die Kletthaken unter dem textilen Material gut verhaken, da sie tief in das textile Substrat eintauchen können. Zur guten Verbindung trägt auch bei, dass den Kletthaken nicht nur die oberflächlichen Schlaufen des Verbundstoffes zur Verfügung stehen, sondern die Kletthaken sich auch in dem Grundgelege des textilen Substrates verhaken können.

In weiterer Ausgestaltung lehrt die Erfindung, dass die Trägerfolie und das textile Substrat miteinander verklebt sind, wobei der Klebstoff in einem Muster appliziert ist, welches sich aus Klebeflächen und klebstofffreien Bereichen zusammensetzt. Die erfindungsgemäße Lehre ermöglicht sehr hohe Verbundhaftungswerte zwischen textilem Substrat und Trägerfolie bei gleichzeitig guter Klettwirkung. Im Bereich der Klebeflächen kann eine große Klebstoffmenge gewählt werden, die eine sichere, kraftschlüssige Verbindung zwischen textilem Substrat und Trägerfolie sicherstellt, wobei auch eine weitgehende Durchtränkung des textilen Materials an den Klebeflächen in Kauf genommen werden kann. Die den Klebeflächen benachbarten klebstofffreien Bereiche bleiben für die Verbindung mit Kletthaken voll erhalten. Die Kletthaken lassen sich an den klebstofffreien Bereichen, in denen Trägerfolie und textiles Substrat lose aufeinander liegen, tief in das textile Substrat einschieben. Zusätzlich zu den oberflächlichen Schlaufen stehen im Bereich der klebstofffreien Bereiche auch die das Grundgelege des textilen Substrats bildenden Garne für eine Verankerung mit Kletthaken zur Verfügung. Die Kletthaken können hinter die Garne des Grundgeleges eingreifen. Der Verbundstoff erhält hierdurch eine Klettwirkung, die signifikant höher ist als bei einer vollflächigen Verklebung von textilem Substrat und Trägerfolie. Die erfindungsgemäße Lehre besteht im Kern darin, dass die zur Verfügung stehende Verbindungsfläche des Kaschierverbundes aufgeteilt wird in Klebeflächen, die ausschließlich oder zumindest primär einen sicheren Verbund zwischen Trägerfolie und textilem Substrat herstellen, und klebstofffreien Bereichen, in denen eine gute Klettwirkung zwischen textilem Substrat und Kletthaken gewährleistet ist. Klebstofffreie Bereiche und Klebeflächen wechseln sich dergestalt ab, dass aus dem Verbundstoff Streifen für Klettverschlüsse mit guten Gebrauchseigenschaften abgetrennt werden können.

Vielfältige Klebstoffmuster zur Verbindung von Trägerfolie und textilem Substrat können im Rahmen der erfindungsgemäßen Lehre verwendet werden. Im Rahmen der Erfindung liegt eine punktförmige Verklebung der Trägerfolie und des textilen Substrats. Ferner besteht die Möglichkeit, dass der Klebstoff ein Muster aus parallelen oder sich kreuzenden Streifen bildet, wobei die Streifen als gerade Linien oder wellenförmige Linien ausgebildet sein können. Eine weitere bevorzugte Ausführung der Erfindung sieht vor, dass der Klebstoff ein Muster mit einer zellenförmigen Struktur bildet, wobei die zellenförmige Struktur offene Zellen mit klebstofffreien Bereichen oder geschlossene Zellen mit Klebeflächen aufweist. Zellenstrukturen mit offenen, klebstofffreien Zellen erweisen sich als besonders wirksam.

Das textile Substrat besteht gemäß einer bevorzugten Ausführung der Erfindung aus einer Kettwirkware, welche ein Grundgelege aus Filamentgarnen und mit dem Grundgelege wirktechnisch verbundene Schlaufen aufweist. Die Kettwirkware weist zweckmäßig ein Flächengewicht zwischen 5g/m² und 50 g/m² auf. Sie ist durchscheinend und sehr luftdurchlässig.

Die erfindungsgemäße Lehre ermöglicht die Verarbeitung von textilen Substraten mit geringem Flächengewicht zu funktionellen Windelverschlüssen. Das textile Substrat kann aus Monofil- und/oder Multifilamentgarnen, beispielsweise aus Polypropylen, Polyester, Polyamid oder anderen textiltechnisch verarbeitbaren Kunststoffen bestehen.

Die Trägerfolie weist gemäß einer bevorzugten Ausführung ebenfalls ein Flächengewicht zwischen 5 und 50 g/m² auf. Es können Monofolien ebenso wie mehrschichtige coextrudierte oder kaschierte Folien verwendet werden. Geeignet sind beispielsweise Folien aus Polyethylen, Polypropylen, Polyester, Polyamid, sowie Mischungen und Copolymerisate auf Basis dieser Polymere. Vorzugsweise werden Trägerfolien verwendet, deren Oberflächen im Schöndruck bedruckt werden können. Es können bedruckte und/oder geprägte Trägerfolien eingesetzt werden.

Für die Verklebung von textilem Substrat und Trägerfolie eignen sich prinzipiell alle auf dem Gebiet der Kaschierfolien verwendeten Klebstoffe. Bevorzugt sind Schmelzklebstoffe auf der Basis PAO, EVA, SBS, SIS, reaktive Polyurethanklebstoffe, Acrylatklebstoffe sowie auch strahlenhärtende Klebstoffe.

Gegenstand der Erfindung ist auch ein Verfahren nach Anspruch 11 zur Herstellung des beschriebenen Verbundstoffes. Bevorzugte Ausgestaltungen des erfindungsgemäßen Verfahrens sind in den Ansprüchen 12 und 13 beschrieben.

Im Folgenden wird die Erfindung anhand einer lediglich ein Ausführungsbeispiel darstellenden Zeichnung erläutert. Es zeigen schematisch:
- **Fig. 1**: einen Längsschnitt durch einen erfindungsgemäßen Verbundstoff für Klettverschlüsse,
- **Fig. 2**: eine Draufsicht auf den in Fig. 1 dargestellten Verbundstoff und dessen Schichten,
- **Fig. 3a bis 3e**: Klebstoffmuster, die eine Verbindungsschicht zwischen Trägerfolie und textilem Substrat des in den Fig. 1 und 2 dargestellten Verbundstoffes bilden,
- **Fig. 4 und 5**: eine Vorrichtung zur Herstellung des Verbundstoffes in verschiedenen Ausführungen.

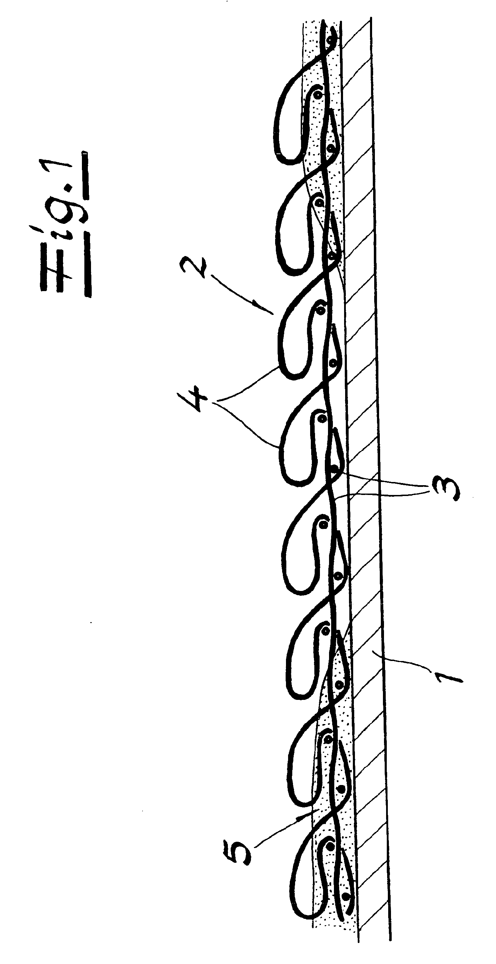

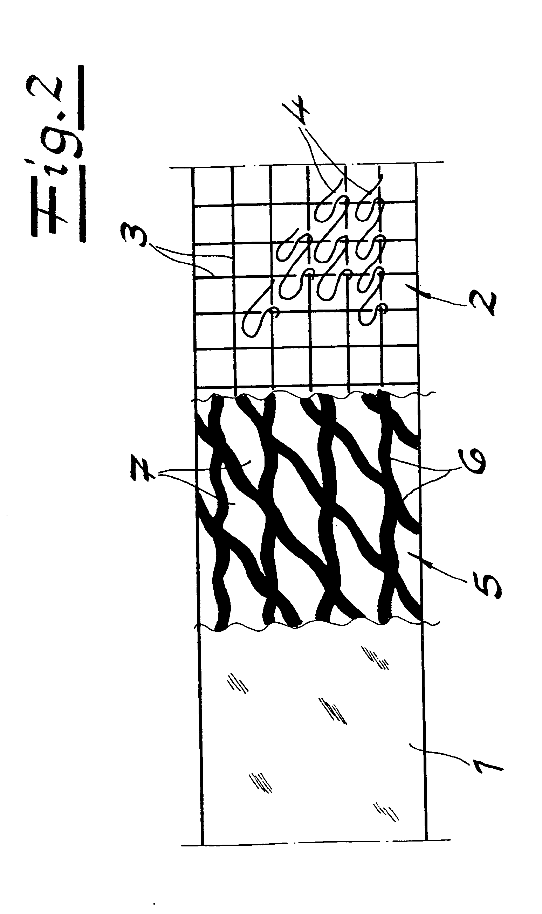

Der in den Fig. 1 und 2 schematisch dargestellte Verbundstoff ist als weiblicher Teil von Klettverschlüssen einsetzbar. Der Verbundstoff ist insbesondere für Klettverschlüsse an Wegwerfprodukten, z. B. Babywindeln, bestimmt. Er besteht in an sich bekannter Weise aus einer Trägerfolie 1 und einem auf die Trägerfolie 1 aufkaschierten textilen Substrat 2, das als Kettwirkware ein Grundgelege 3 aus Filamentgarnen sowie mit dem Grundgelege 3 wirktechnisch verbundene Schlaufen 4 aufweist. Die Schlaufen 4 sind oberflächlich angeordnet und zur Verbindung mit nicht dargestellten Kletthaken eines Verschlussbandes geeignet.

Die Trägerfolie 1 und das textile Substrat 2 sind nicht vollflächig verbunden. Die Fläche, an der die Trägerfolie 1 mit dem textilen Substrat 2 verbunden ist, beträgt vorzugsweise 20 bis 80 % der kaschierten Folienfläche.

Den Darstellungen in den Fig. 1 und 2 entnimmt man, dass die Trägerfolie 1 und das textile Substrat 2 miteinander verklebt sind, wobei der die Verbindungsschicht 5 bildende Klebstoff in einem Muster appliziert ist, welche sich aus Klebeflächen 6 und klebstofffreien Bereichen 7 zusammensetzt. An den Klebeflächen 6 ist das textile Substrat 2 durch einen variabel festlegbaren Klebstoffauftrag 8 fest an der Trägerfolie 1 verankert. Der Fig. 1 entnimmt man, dass die Filamente des textilen Substrates 2 im Klebstoff, beispielsweise einem Schmelzklebstoff, eingesunken sind und von diesem umschlossen werden. Dabei kann in Kauf genommen werden, dass auch Schlaufen im Bereich der Klebeflächen 6 teilweise mit verklebt werden und im Bereich der Klebeflächen 6 nur wenig wirksam sind. In den klebstofffreien Bereichen 7 liegen die Trägerfolie 1 und das textile Substrat 2 lose aufeinander. Kletthaken lassen sich in den klebstofffreien Bereichen 7 des Verbundstoffes tief in das textile Substrat 2 einschieben, wobei nicht nur eine Verhakung an den oberflächlichen freien Schlaufen 4 erfolgt, sondern zusätzlich auch die Filamentgarne des Grundgeleges 3 für die Verankerung der Kletthaken zur Verfügung stehen. Die Kletthaken greifen hinter die Filamentgarne des textilen Substrates 2. Die klebstofffreien Bereiche 7 des Verbundstoffes zeichnen sich durch eine große Klettwirkung aus und verleihen dem Verbundstoff gute Gebrauchseigenschaften.

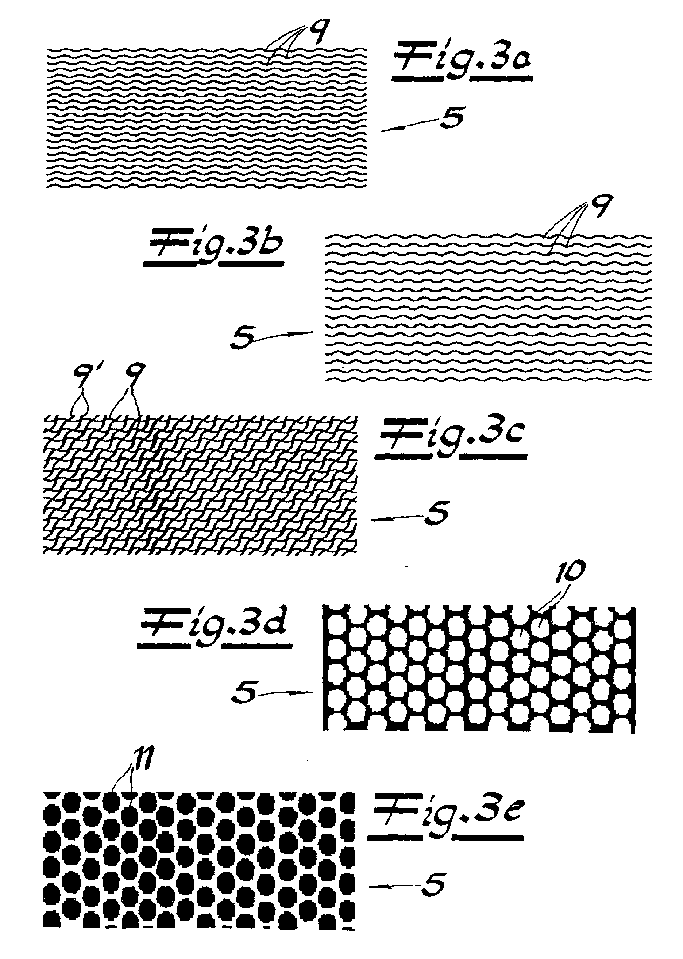

Die Fig. 3a bis 3e zeigen verschiedene Klebstoffmuster der erfindungsgemäßen Verbindungsschicht 5 zwischen Trägerfolie und textilem Substrat. In den Ausführungsbeispielen 3a und 3b bildet der Klebstoff ein Muster aus parallelen, wellenlinienförmig verlaufenden Streifen 9. Die in den Fig. 3a und 3b dargestellten Muster unterscheiden sich durch den Abstand der Klebstoffstreifen 9 zueinander. Im Ausführungsbeispiel der Fig. 3c bildet der Klebstoff ein Muster aus sich kreuzenden Streifen 9, 9'. Die Fig. 3d zeigt ein Klebstoffmuster mit einer zellenförmigen Struktur, wobei die zellenförmige Struktur offene Zellen 10 mit klebstofffreien Bereichen aufweist. In dem mit Fig. 3e dargestellten Ausführungsbeispiel bildet der Klebstoff ebenfalls ein Muster mit einer zellenförmigen Struktur, wobei die zellenförmige Struktur jedoch geschlossene Zellen 11 mit Klebeflächen aufweist. Es resultiert eine punktförmige Verklebung der Trägerfolie 1 und des textilen Substrates 2, wobei die Anzahl der Klebepunkte pro Flächeneinheit anhand weniger Versuche festgelegt werden kann. In allen Ausführungsbeispielen sollten die Klebeflächen nicht weniger als 20 % und maximal 80 % der kaschierten Fläche betragen.

Das textile Substrat weist vorzugsweise ein Flächengewicht zwischen 5 g/m² und 50 g/m² auf. Es ist luftdurchlässig und durchscheinend. Die Trägerfolie kann aufgrund der Durchlässigkeit des textilen Substrats bedruckt werden. Als Trägerfolien sind Folien z B. aus Polyolefinen, Polyester, Polyamid, Mischungen oder Copolymerisaten dieser Polymere einsetzbar. Sie weisen vorzugsweise ebenfalls ein Flächengewicht zwischen 5 und 50 g/m² auf.

Zur Verklebung der Trägerfolie mit dem textilen Substrat können beispielsweise Schmelzklebstoffe auf der Basis PAO, EVA, SBS, SIS, reaktive Polyurethanklebstoffe, Acrylatklebstoffe sowie auch strahlenhärtende Klebstoffe eingesetzt werden. Die Klebstoffmengen werden auf die Klebeflächen so abgestimmt, dass ein fester Verbund zwischen Trägerfolie und textilem Substrat resultiert. Daher sind die Klebstoffauftragsmengen anwendungsabhängig variabel festlegbar.

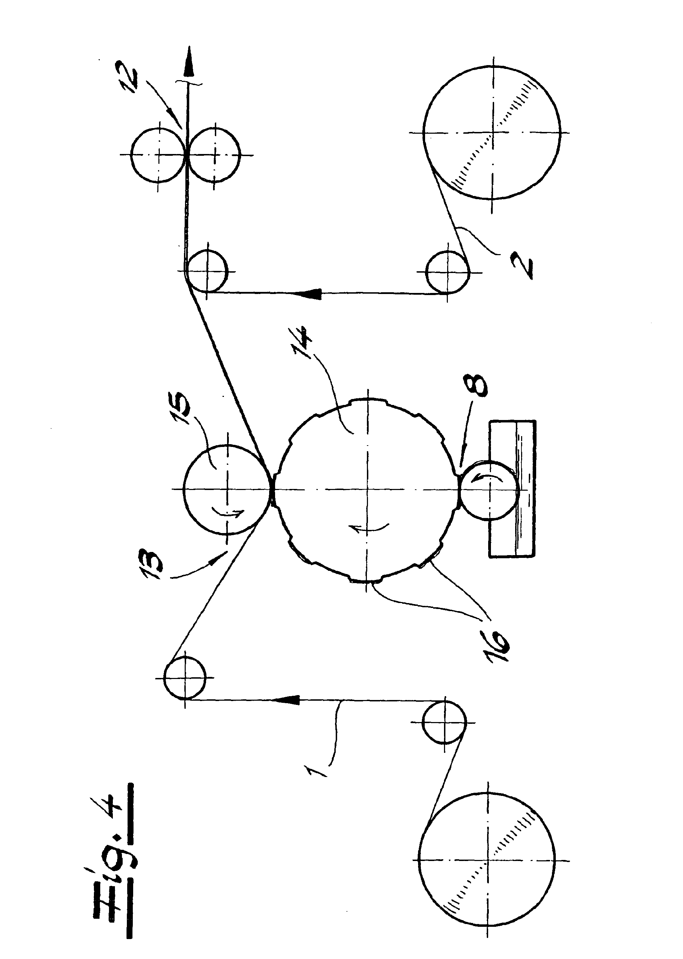

Das Verfahren zur Herstellung des Verbundstoffes ist in Fig. 4 schematisch dargestellt. Auf eine Trägerfolie 1 wird Klebstoff in einem Muster appliziert, welches sich aus Klebeflächen und klebstofffreien Bereichen zusammensetzt. Eine Materialbahn aus einem textilen Substrat 2, welches zur Verbindung mit Kletthaken geeignete oberflächliche Schlaufen aufweist, wird auf die mit Klebstoff versehene Seite der Trägerfolie 1 aufgebracht. Die dabei gebildete zweilagige Bahn durchläuft den Walzenspalt eines Walzenpaares 12, in dem die Trägerfolie 1 mit dem textilen Substrat 2 verpresst wird.

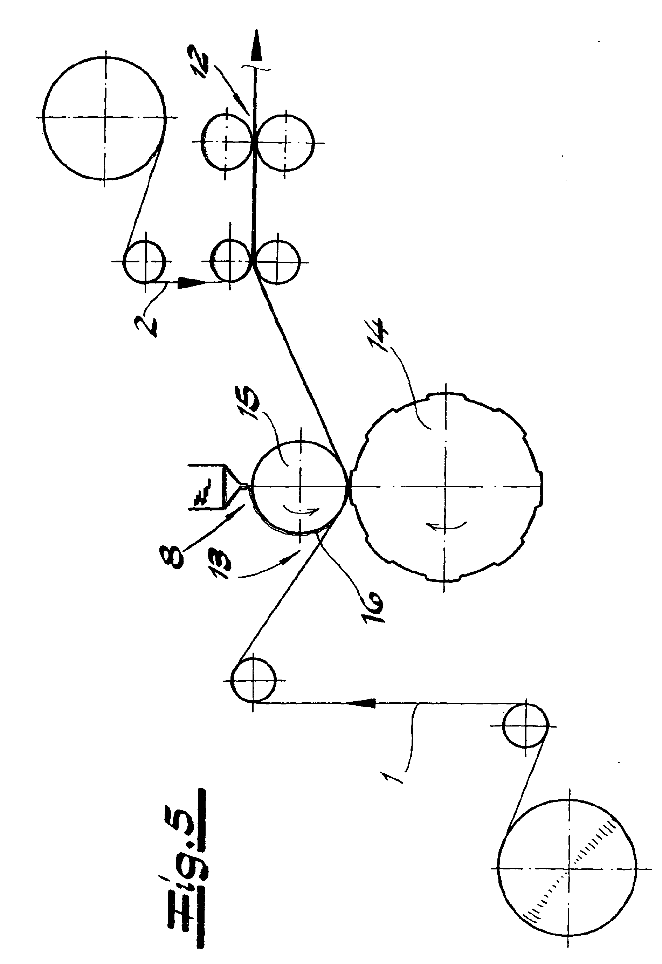

Der Klebstoff kann durch Düsenauftragsverfahren, durch eine Sprühtechnik und ähnliche Verfahren auf die Trägerfolie aufgebracht werden. Bei der in den Ausführungsbeispielen dargestellten bevorzugten Ausführung des Verfahrens wird der Klebstoff nach einem Rotationsdruckverfahren auf die Trägerfolie 1 aufgebracht. Hierbei durchläuft die Trägerfolie 1 eine Druckwalzenanordnung 13 bestehend aus einem Gravurzylinder 14 und einem die Folie an den Gravurzylinder andrückenden Gegenzylinder, wobei die Oberfläche des Gravurzylinders 14 mit einer dem Klebstoffmuster entsprechenden Gravur versehen ist. Bei der in Fig. 4 dargestellten Ausgestaltung des Verfahrens wird auf die Oberfläche des Gravurzylinders 14 ein Klebstofffilm 16 aufgebracht, der an den erhabenen Flächen der Gravur auf die Trägerfolie 1 übertragen wird. Bei der in Fig. 5 dargestellten Verfahrensvariante wird der Klebstofffilm 16 auf die Oberfläche des Gegenzylinders 15 aufgebracht. Der Gravurzylinder 14 presst die Trägerfolie 1 jedoch ausschließlich in den erhabenen Bereichen der Gravur gegen den Klebstofffilm 16 an der Oberfläche des Gegenzylinders 15, so dass auch bei dieser Ausgestaltung der Klebstofffilm lediglich an den erhabenen Flächen der Gravur auf die Trägerfolie übertragen wird.

### Beispiele:

Die für die Funktionalität eines Klettverschlusses entscheidenden Eigenschaften werden durch drei Messgrößen charakterisiert. Maßgebliche Messgrößen sind Öffnungskraft, Scherkraft sowie Verbundhaftung zwischen textilem Substrat und Trägerfolie. Unter Öffnungskraft versteht man die Kraft, die aufzuwenden ist, um Kletthaken eines Klettverschlusses aus dem textilen Substrat zu lösen. Die Öffnungskraft wird durch einen Zugversuch ermittelt, wobei der manuelle Öffnungsvorgang durch eine Zugkraftmessung am Klettverschluss in Öffnungsrichtung simuliert wird. Als maximale Scherkraft wird die Kraft bezeichnet, bei der sich die Verbindung zwischen Kletthaken und textilem Substrat aufgrund zu hoher Last ungewollt löst. Auch diese Kraft wird durch eine Zugkraftmessung unter Simulation der bei der Handhabung auftretenden Zugbeanspruchung gemessen. Die Verbundhaftung zwischen textilem Substrat und Trägerfolie wird in einem Schälversuch durch Abziehen des textilen Substrats von der Trägerfolie ermittelt.

Es wurden Verbundstoffe nach den in den Fig. 4 und 5 dargestellten Verfahren hergestellt. Das textile Substrat und die Trägerfolie wurden nicht verändert. Variiert wurden die Klebstoffauftragsmenge und das Klebstoffmuster zur Verbindung von Trägerfolie und textilem Substrat. Es wurden Klebstoffmuster entsprechend den Fig. 3a bis 3c gewählt. In einem Vergleichsversuch erfolgte eine vollflächige Verklebung der Trägerfolie mit dem textilen Substrat. Gemessen wurden das Flächengewicht des Verbundstoffes, welches von der Klebstoffauftragsmenge geringfügig abhängig ist, die Verbundhaftung, Öffnungskraft und Scherkraft. Die Messungen wurden an Proben mit einer Klettfläche von 25,4 mm Länge und 13 mm Breite durchgeführt. Die Versuchsergebnisse sind in Tabelle 1 dargestellt. Die nach dem erfindungsgemäßen Verfahren hergestellten Verbundstoffe zeichnen sich durch eine große Verbundhaftung sowie gute Werte der Öffnungskraft und Scherfestigkeitswerte aus.

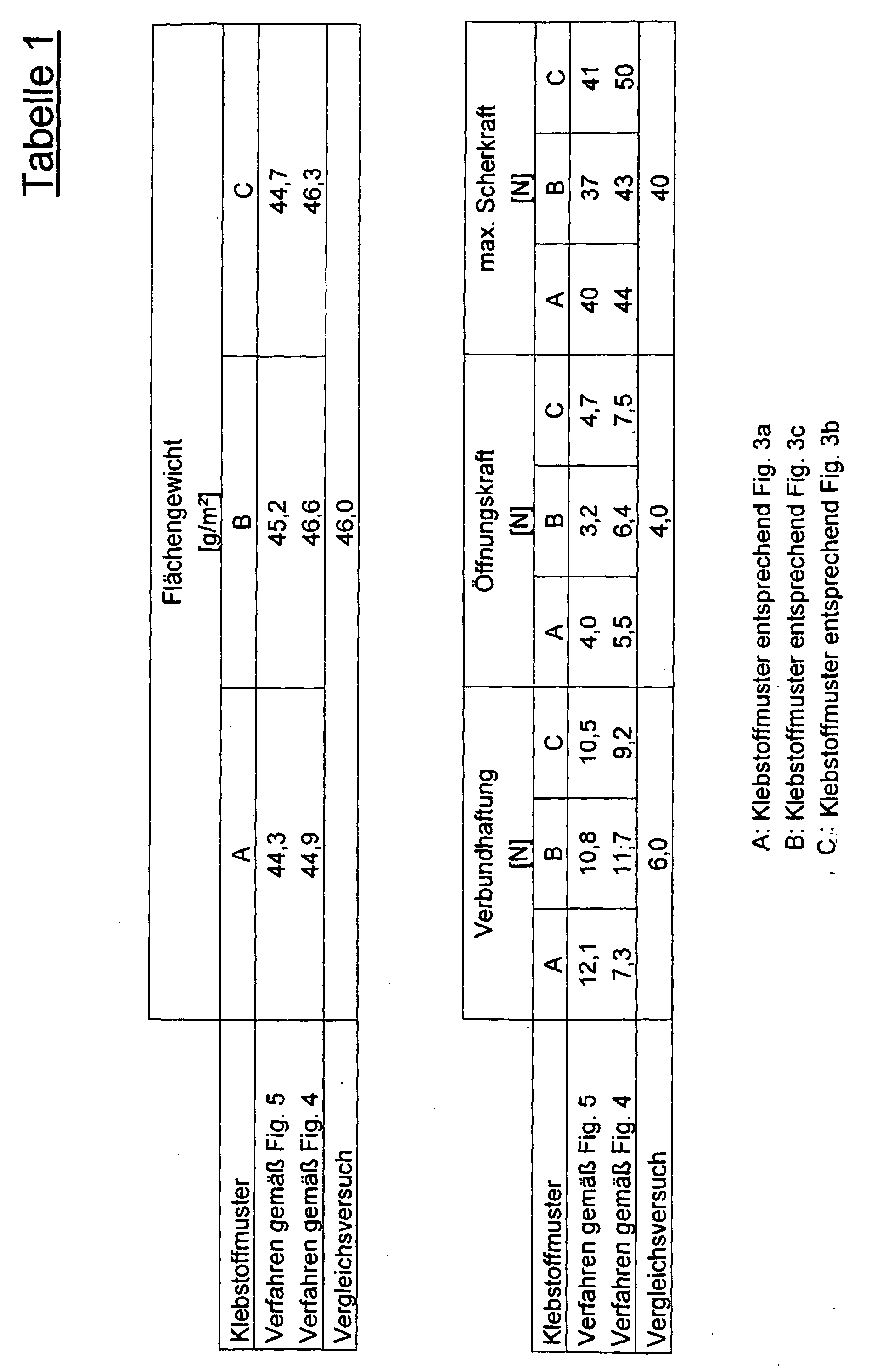

## Patentansprüche

1. Verbundstoff für Klettverschlüsse, insbesondere Windelverschlüsse, mit einer Trägerfolie (1) und einem auf die Trägerfolie (1) aufkaschierten textilen Substrat (2), welches oberflächliche, zur Verbindung mit Kletthaken geeignete Schlaufen (4) aufweist, **dadurch gekennzeichnet, dass** die Trägerfolie (1) und das textile Substrat (2) nicht vollflächig verbunden sind.

2. Verbundstoff nach Anspruch 1, **dadurch gekennzeichnet, dass** die Fläche, an der die Trägerfolie (1) mit dem textilen Substrat (2) verbunden sind, 20 % bis 80 % der kaschierten Folienfläche beträgt.

3. Verbundstoff nach Anspruch 1 oder 2, **dadurch gekennzeichnet, dass** die Trägerfolie (1) und das textile Substrat (2) miteinander verklebt sind, wobei der Klebstoff in einem Muster appliziert ist, welches sich aus Klebeflächen (6) und klebstofffreien Bereichen (7) zusammensetzt.

4. Verbundstoff nach Anspruch 3, **dadurch gekennzeichnet, dass** die Trägerfolie und das textile Substrat (2) punktförmig verklebt sind.

5. Verbundstoff nach Anspruch 3, **dadurch gekennzeichnet, dass** der Klebstoff ein Muster aus parallelen oder sich kreuzenden Streifen (9, 9') bildet.

6. Verbundstoff nach Anspruch 3, **dadurch gekennzeichnet, dass** der Klebstoff ein Muster mit einer zellenförmigen Struktur bildet, wobei die zellenförmige Struktur offene Zellen (10) mit klebstofffreien Bereichen oder geschlossene Zellen mit Klebeflächen aufweist.

7. Verbundstoff nach einem der Ansprüche 1 bis 6, **dadurch gekennzeichnet, dass** das textile Substrat (2) aus einer Kettwirkware besteht, welches ein Grundgelege (3) aus Filamentgarnen und mit dem Grundgelege (3) wirktechnische verbundene Schlaufen (4) aufweist.

8. Verbundstoff nach einem der Ansprüche 1 bis 7, **dadurch gekennzeichnet, dass** das textile Substrat (2) ein Flächengewicht von 5 g/m² bis 50 g/m² aufweist.

9. Verbundstoff nach einem der Ansprüche 1 bis 8, **dadurch gekennzeichnet, dass** die Trägerfolie (1) bedruckt und/oder geprägt ist.

10. Verbundstoff nach einem der Ansprüche 1 bis 9, **dadurch gekennzeichnet, dass** die Trägerfolie (1) ein Flächengewicht von 5 bis 50 g/m² aufweist.

11. Verfahren zur Herstellung eines Verbundstoffes für Klettverschlüsse nach einem der Ansprüche 1 bis 10,
wobei auf eine Trägerfolie Klebstoff in einem Muster appliziert wird, welches sich aus Klebeflächen und klebstofffreien Bereichen zusammensetzt,
wobei eine Materialbahn aus einem textilen Substrat, welches zur Verbindung mit Kletthaken geeignete oberflächliche Schlaufen aufweist, auf die mit Klebstoff versehene Seite der Trägerfolie aufgebracht wird und
wobei die dabei gebildete zweilagige Bahn den Walzenspalt eines Walzenpaares durchläuft, in dem die Trägerfolie mit dem textilen Substrat verpresst wird.

12. Verfahren nach Anspruch 11, **dadurch gekennzeichnet, dass** der Klebstoff nach einem Rotationsdruckverfahren auf die Trägerfolie aufgebracht wird.

13. Verfahren nach Anspruch 12, **dadurch gekennzeichnet, dass** die Trägerfolie eine Druckwalzenanordnung bestehend aus einem Gravurzylinder und einem die Folie an den Gravurzylinder andrückenden Gegenzylinder durchläuft, wobei die Oberfläche des Gravurzylinders mit einer dem Klebstoffmuster entsprechenden Gravur versehen ist und wobei auf die Oberfläche des Gravurzylinders oder die Oberfläche des Gegenzylinders ein Klebstofffilm aufgebracht wird, der an den erhabenen Flächen der Gravur auf die Trägerfolie übertragen wird.

## Claims

1. Composite material for hook-and-loop fasteners, in particular nappy fasteners, comprising a carrier film (1) and a textile substrate (2) laminated onto the carrier film (1), which textile substrate has surface loops (4) which are suitable for connecting to hooks, **characterised in that** the carrier film (1) and the textile substrate (2) are not connected over their entire surface.

2. Composite material according to Claim 1, **characterised in that** the surface area over which the carrier film (1) is connected to the textile substrate (2) represents 20% to 80% of the laminated film surface.

3. Composite material according to Claim 1 or 2, **characterised in that** the carrier film (1) and the textile substrate (2) are adhesively bonded to one another, wherein the adhesive is applied in a pattern which is composed of adhesive areas (6) and adhesive-free areas (7).

4. Composite material according to Claim 3, **characterised in that** the carrier film and the textile substrate (2) are adhesively bonded at points.

5. Composite material according to Claim 3, **characterised in that** the adhesive forms a pattern of parallel or intersecting strips (9, 9').

6. Composite material according to Claim 3, **characterised in that** the adhesive forms a pattern with a cellular structure, wherein the cellular structure has open cells (10) comprising adhesive-free areas or closed cells comprising adhesive areas.

7. Composite material according to one of Claims 1 to 6, **characterised in that** the textile substrate (2) consists of a warp-knitted fabric which comprises a basic interlaid scrim (3) of filament yarns and loops (4) which are connected to the basic interlaid scrim (3) by means of knitting technology.

8. Composite material according to one of Claims 1 to 7, **characterised in that** the textile substrate (2) has a basis weight of 5 g/m² to 50 g/m².

9. Composite material according to one of Claims 1 to 8, **characterised in that** the carrier film (1) is printed and/or embossed.

10. Composite material according to one of Claims 1 to 9, **characterised in that** the carrier film (1) has a basis weight of 5 to 50 g/m².

11. Method for producing a composite material for hook-and-loop fasteners according to one of Claims 1 to 10,
wherein adhesive is applied to a carrier film in a pattern which is composed of adhesive areas and adhesive-free areas,
wherein a material web of a textile substrate, which has surface loops which are suitable for connecting to hooks, is applied to the side of the carrier film provided with adhesive, and
wherein the two-ply web thus formed runs through the nip of a pair of rollers, in which the carrier film is pressed to the textile substrate.

12. Method according to Claim 11, **characterised in that** the adhesive is applied to the carrier film according to a rotary printing process.

13. Method according to Claim 12, **characterised in that** the carrier film runs through a printing roller arrangement consisting of a gravure cylinder and a counter-cylinder which presses the film against the gravure cylinder, wherein the surface of the gravure cylinder is provided with a gravure which corresponds to the adhesive pattern, and wherein an adhesive film is applied to the surface of the gravure cylinder or to the surface of the counter-cylinder, which adhesive film is transferred to the carrier film at the raised areas of the gravure.

## Revendications

1. Matière composite pour des fermetures à griffes, notamment des attaches de couches à langer, comportant un film de support (1) et un substrat textile (2) marouflé sur le film de support (1), ce substrat ayant des bouclettes (4) en surface, pour être reliées à des griffes,
**caractérisée en ce que**
le film de support (1) et le substrat textile (2) ne sont pas reliés sur toute leur surface.

2. Matière composite selon la revendication 1,
**caractérisée en ce que**
la surface de liaison du film de support (1) au substrat textile (2) représente une surface de film marouflée de 20 % à 80 %.

3. Matière composite selon la revendication 1 ou 2,
**caractérisée en ce que**
le film de support (1) et le substrat textile (2) sont collés et la colle est appliquée suivant un motif composé de zones de surfaces encollées (6) et de zones sans colle (7).

4. Matière composite selon la revendication 3,
**caractérisée en ce que**
le film de support et le substrat textile (2) sont collés ponctuellement.

5. Matière composite selon la revendication 3,
**caractérisée en ce que**
la colle forme un motif de bandes parallèles ou croisées (9, 9').

6. Matière composite selon la revendication 3,
**caractérisée en ce que**
la colle forme un motif à structure cellulaire, cette structure ayant des cellules ouvertes (10) avec des zones sans colle et des cellules fermées avec des surfaces encollées.

7. Matière composite selon l'une des revendications 1 à 6,
**caractérisée en ce que**
le substrat textile (2) se compose d'une structure de base en fil continu et d'une structure de base avec des bouclettes (4) liées par tricotage.

8. Matière composite selon l'une des revendications 1 à 7,
**caractérisée en ce que**
le substrat textile (2) a un poids surfacique de 5 g/m² jusqu'à 50 g/m².

9. Matière composite selon l'une des revendications 1 à 8,
**caractérisée en ce que**
le film de support (2) est imprimé et/ ou matricé.

10. Matière composite selon l'une des revendications 1 à 9,
**caractérisée en ce que**
le film de support (1) a un poids surfacique compris entre 5 et 50 g/m².

11. Procédé de fabrication d'une matière composite pour des liaisons à griffes selon l'une des revendications 1 à 10,
selon lequel
on applique de la colle suivant un motif sur le film de support, ce motif se composant de zones encollées et de zones sans colle,
on applique une bande de matière formée d'un substrat textile ayant des bouclettes en surface, destinées à être reliées à des griffes, sur la face du film de support, munie de colle et
on fait passer la nappe à deux couches ainsi formée dans l'intervalle d'une paire de cylindres pour comprimer le film de support au substrat textile.

12. Procédé selon la revendication 11,
**caractérisé en ce qu'**
on applique la colle par un procédé d'impression par rotation sur le film de support.

13. Procédé selon la revendication 12,
**caractérisé en ce que**
le film de support traverse un dispositif à cylindres d'impression composé d'un cylindre gravé et d'un contre-cylindre appliquant le film contre le cylindre gravé, la surface du cylindre gravé, ayant une gravure selon le motif d'encollage et la surface du cylindre gravé ou celle du contre-cylindre reçoit un film de colle que les surfaces en relief de la gravure transfèrent au film de support.
